# EUROPÄISCHE PATENTSCHRIFT

(11) **EP 0 693 071 B1**
(45) Veröffentlichungstag und Bekanntmachung des Hinweises auf die Patenterteilung: **10.12.1997**
(21) Anmeldenummer: 94912542.1
(22) Anmeldetag: 24.03.1994
(51) Int. Cl.: C07D 473/32, A61K 31/52

(54) **NEUE CARBONSÄUREESTER VON 2-AMINO-7-(1,3-DIHYDROXY-2-PROPOXYMETHYL)PURIN, DEREN HERSTELLUNG SOWIE DEREN VERWENDUNG**
NEW CARBOXYLIC-ACID ESTERS OF 2-AMINO-7-(1,3-DIHYDROXY-2-PROPOXYMETHYL)PURINE, THE PREPARATION OF SUCH ESTERS AND THEIR USE
NOUVEAUX ESTERS CARBOXYLIQUES DE 2-AMINO-7-(1,3-DIHYDROXY-2-PROPOXYMETHYL)PURINE, LEUR FABRICATION ET LEUR UTILISATION

(30) Priorität: 09.04.1993 DE 4311801
(43) Veröffentlichungstag der Anmeldung: 24.01.1996
(73) Patentinhaber: HOECHST AKTIENGESELLSCHAFT, 65929 Frankfurt am Main (DE)
(72) Erfinder: JÄHNE, Gerhard, D-65929 Frankfurt am Main (DE); HELSBERG, Matthias, D-65779 Kelkheim (DE); WINKLER, Irvin, D-65835 Liederbach (DE); GROSS, Gerhard, D-65439 Flörsheim am Main (DE); SCHOLL, Thomas, D-53113 Bonn (DE)
(86) Internationale Anmeldenummer: EP9400951
(87) Internationale Veröffentlichungsnummer: WO9424134

(56) Entgegenhaltungen:
- EP-A- 0 452 680
- EP-A- 0 563 814

## Beschreibung

Es wurde bereits gefunden (s. EP 452 680), daß substituierte Purine der Formel 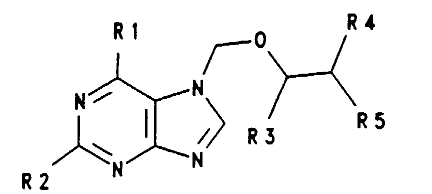 eine antivirale Wirkung aufweisen.

Es hat sich nun herausgestellt, daß bestimmte Carbonsäureester substituierter Purine eine besondere antivirale Wirksamkeit haben und darüber hinaus eine besonders ausgeprägte Bioverfügbarkeit nach oraler Verabreichung aufweisen.

Erfindungsgegenstand sind demzufolge Verbindungen der Formel 1 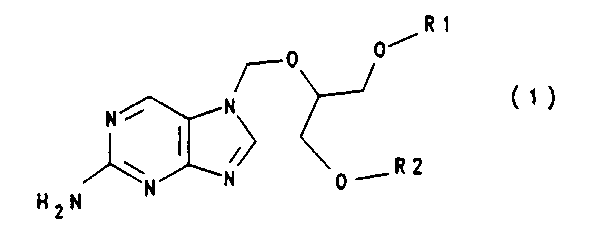 in denen die Reste R¹ und/oder R² unabhängig voneinander Acylreste der Formel

- C ( = O ) - R³ (2)

darstellen,
wobei R³ C1-C5-Alkoxymethyl, C4-C17-Alkyl, C6-C17-Alkenyl, C3-C8-Cycloalkyl, Aryl mit 6-14 C-Atomen, vorzugsweise 6 C-Atomen gegebenenfalls substituiert mit C1-C3-Alkyl oder Methoxy; Heteroaryl mit 3 bis 10 C-Atomen, vorzugsweise 4 oder 5 C-Atomen, mit bis zu 3 Heteroatomen, ausgewählt aus der Gruppe O, N, und S, vorzugsweise mit einem oder zweien, insbesondere einem O- oder N-Atom; Arylmethyl mit Aryl wie vorstehend definiert, Heteroarylmethyl mit Heteroaryl wie vorstehend definiert bedeutet und einer der Reste R¹ und R² auch Wasserstoff bedeuten kann, sowie deren physiologisch verträgliche Salze.

Der vorstehend unter R³ definierte Alkoxymethylrest kann geradkettig oder verzweigt sein. Beispiele sind Methoxymethyl, Ethoxymethyl, Propoxymethyl oder Isopropoxymethyl.
Der vorstehend unter R³ definierte Alkylrest kann geradkettig oder verzweigt sein.
Beispiele sind Butyl, Pentyl, Hexyl, Heptyl, Hexadecyl oder Heptadecyl.
Der vorstehend unter R³ definierte Alkenylrest kann geradkettig oder verzweigt sein. Beispiele sind Oct-3-enyl, Non-5-enyl, Dec-7-enyl, 2,4,4-Trimethylpent-2-enyl oder Heptadec-9-enyl.
Beispiele für den vorstehend definierten Cycloalkylrest sind Cyclopentyl, Cyclohexyl oder Cycloheptyl.
Der vorstehend unter R³ definierte Arylrest bzw. der Arylrest von Arylmethyl ist beispielsweise Phenyl oder Naphthyl.

Der vorstehend unter R³ definierte Heteroarylrest bzw. der Heteroarylrest von Heteroarylmethyl ist beispielsweise Thiophenyl, Pyridyl oder Furyl.

Eine besondere Bedeutung besitzen Verbindungen der Formel 1 wie zuvor beschrieben, in denen
R³ C1-C3-Alkoxymethyl, C4-C17-Alkyl, C13-C17-Alkenyl, C5-C6-Cycloalkyl, Phenyl, Thiophen-3-yl, Benzyl oder Pyridin-3-yl-methyl bedeutet und einer der Reste R¹ und R² auch Wasserstoff bedeuten kann.

Eine ganz besondere Bedeutung besitzen die obengenannten Verbindungen der Formel 1, in denen R³ C1-C2-Alkoxymethyl, C17-Alkyl, C17-Alkenyl, C6-Cycloalkyl, Phenyl, Thiophen-3-yl, Benzyl oder Pyridin-3-yl-methyl bedeutet.

Die erfindungsgemäßen Verbindungen der Formel 1 können in der acyclischen Seitenkette ein oder mehrere chirale Zentren aufweisen. Die Verbindungen liegen in der Regel als Racemate vor; eine Herstellung bzw. Isolierung der reinen Enantiomeren ist möglich. Gegenstand der Erfindung sind deshalb sowohl die reinen Enantiomeren als auch Mischungen derselben, wie z. B. das zugehörige Racemat.

Besonders für therapeutische Zwecke geeignete Salze der erfindungsgemäßen Verbindungen sind Salze von physiologisch verträglichen Säuren wie Essigsäure, Milchsäure, Äpfelsäure, p-Toluolsulfonsäure, Methansulfonsäure, Isäthionsäure, Salzsäure, Schwefelsäure oder Phosphorsäure.

Weiterhin gehört zum Gegenstand der vorliegenden Erfindung ein Verfahren zur Herstellung von erfindungsgemäßen Verbindungen, das dadurch gekennzeichnet ist, daß man die Verbindung der Formel 1' 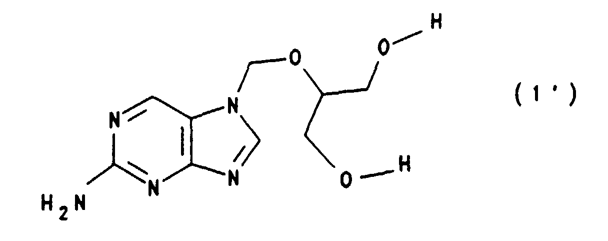 mit Carbonsäuren der Formel 3,

R⁴ - COOH (3)

wobei R⁴ C1-C5-Alkoxymethyl, C4-C17-Alkyl, C6-C17-Alkenyl, C3-C8-Cycloalkyl, Aryl, Heteroaryl, Arylmethyl oder Heteroarylmethyl, wobei Aryl, Heteroaryl, Arylmethyl und Heteroarylmethyl wie vorstehend definiert sind, bedeutet, mit einem wasserentziehenden Mittel unter Beifügung einer Base in einem organischen Lösungsmittel unter Erhalt von Verbindungen der Formel 1 umsetzt und anschließend die Mono- und Diester voneinander mittels herkömmlicher Methoden trennt.

Die obengenannte Verbindung der Formel 1' läßt sich z. B. wie in der EP 452 680 beschrieben, herstellen.
Ein bevorzugtes wasserentziehendes Mittel zur Durchführung der obengenannten Reaktion ist z. B. Dicyclohexylcarbodiimid. Als Basen eignet sich z. B. N,N-Dimethylaminopyridin bzw. dessen Derivate.

Geeignete organische Lösungsmittel zur Durchführung der Reaktion sind z. B. Dimethylformamid, N-Methyl-2-pyrrolidon, Pyridin, Dichlormethan, 1,2-Dichlorethan, Essigsäureethylester, Toluol oder Tetrahydrofuran.

Zur Durchführung der Reaktion wird vorzugsweise die Verbindung der Formel 1' in dem organischen Lösungsmittel gelöst oder suspendiert und vorzugsweise bei -10°C bis +40°C insbesondere bei Raumtemperatur, unter Rühren nacheinander vorzugsweise mit mindestens 2 Äquivalenten insbesondere 2,2 bis 3 Äquivalenten, der entsprechenden Carbonsäure, gegebenenfalls in dem entsprechenden Lösungsmittel gelöst, katalytischen Mengen, vorzugsweise 0,2 bis 0,4 Äquivalenten, N,N-Dimethylaminopyridin, und vorzugsweise wenigstens 2 Äquivalenten, insbesondere 3 bis 5 Äquivalenten, Dicyclohexylcarbodiimid versetzt und anschließend die Mischung 1 bis 24 Stunden, vorzugsweise 6 bis 10 Stunden, bei -10°C bis +50°C, vorzugsweise bei Raumtemperatur bis +40°C, gerührt. Zur Vervollständigung der Reaktion werden gegebenenfalls nach dieser Zeit ein weiteres Äquivalent der entsprechenden Carbonsäure, 0,2 Äquivalente N,N-Dimethylaminopyridin und 2 Äquivalente Dicyclohexylcarbodiimid zugegeben und weitere 5 bis 24 Stunden, vorzugsweise 5 bis 10 Stunden gerührt. Danach wird vom ausgefallenen Dicyclohexylharnstoff abfiltriert und das erfindungsgemäße Produkt isoliert. Die Isolation erfolgt z. B. chromatographisch oder durch Kristallisation, vorzugsweise - nach Einengen des Filtrats - durch Chromatographie z. B. über Kieselgel mit z. B. Dichlormethan/Methanol 9/1.

Die Monoester lassen sich gewinnen, indem man die Reaktion dünnschichtchromatographisch verfolgt und vorzeitig abbricht und danach wie oben beschrieben z. B. chromatographisch reinigt.

Die gegebenenfalls nötige Isolierung von optisch aktiven Verbindungen erfolgt nach Methoden des Standes der Technik.

Zum Erfindungsgegenstand gehört weiterhin die Verwendung der erfindungsgemäßen Verbindungen der Formel 1 als antivirale Mittel zur Behandlung oder Prophylaxe viraler Erkrankungen.

Besonders wirksam sind die erfindungsgemäßen Verbindungen gegen Herpes Simplex Virus Typ 1, Herpes Simplex Virus Typ 2, humanes Cytomegalie-Virus, murines Cytomegalie-Virus, Varicella Zoster Virus, Epstein Barr Virus und humanes Herpes Virus 6 (HHV-6).

Des weiteren betrifft die vorliegende Erfindung Arzneimittel mit einem Gehalt an mindestens einer erfindungsgemäßen Verbindung.

Die erfindungsgemäßen Arzneimittel können bevorzugt enteral (oral) aber auch parenteral (intravenös), rektal oder lokal (topisch) angewendet werden. Sie können in Form von Lösungen, Pulvern, Tabletten, Kapseln (einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe und Streckmitte;, Lösemittel, Emulgatoren, Gleitstoffe, Geschmackskorrigentien, Farbstoffe und/oder Puffersubstanzen in Frage. Als zweckmäßige Dosierung werden 0,1 bis 10, vorzugsweise 0,2 bis 8 mg/kg Körpergewicht verabreicht. Sie werden zweckmäßig in Dosierungseinheiten verabreicht, die mindestens die wirksame Tagesmenge der erfindungsgemäßen Verbindungen z. B. 30 bis 300, vorzugsweise 50 bis 250 mg enthalten.

Wirkung gegen systemische Herpes simplex 1 Virus (HSV-1) Infektion: NMRI-Mäuse, spezifisch pathogenfrei, im Gewicht von 15 bis 18 g wurden intraperitoneal mit HSV-1 infiziert und anschließend mit den erfindungsgemäßen Verbindungen oral therapiert. Die Behandlung erfolgte erstmals 3 Stunden nach der Infektion und wurde zweimal täglich über 4 Tage fortgesetzt. Der Behandlungserfolg wurde anhand des Krankheitsverlaufes und der Überlebensrate gegenüber der unbehandelten Infektionskontrolle bestimmt. Letztere erhielt anstelle der erfindungsgemäßen Verbindungen physiologische Kochsalzlösung appliziert. Die Beobachtungszeit betrug zwei Wochen. Tabelle 1 zeigt die Ergebnisse dieser Untersuchung.

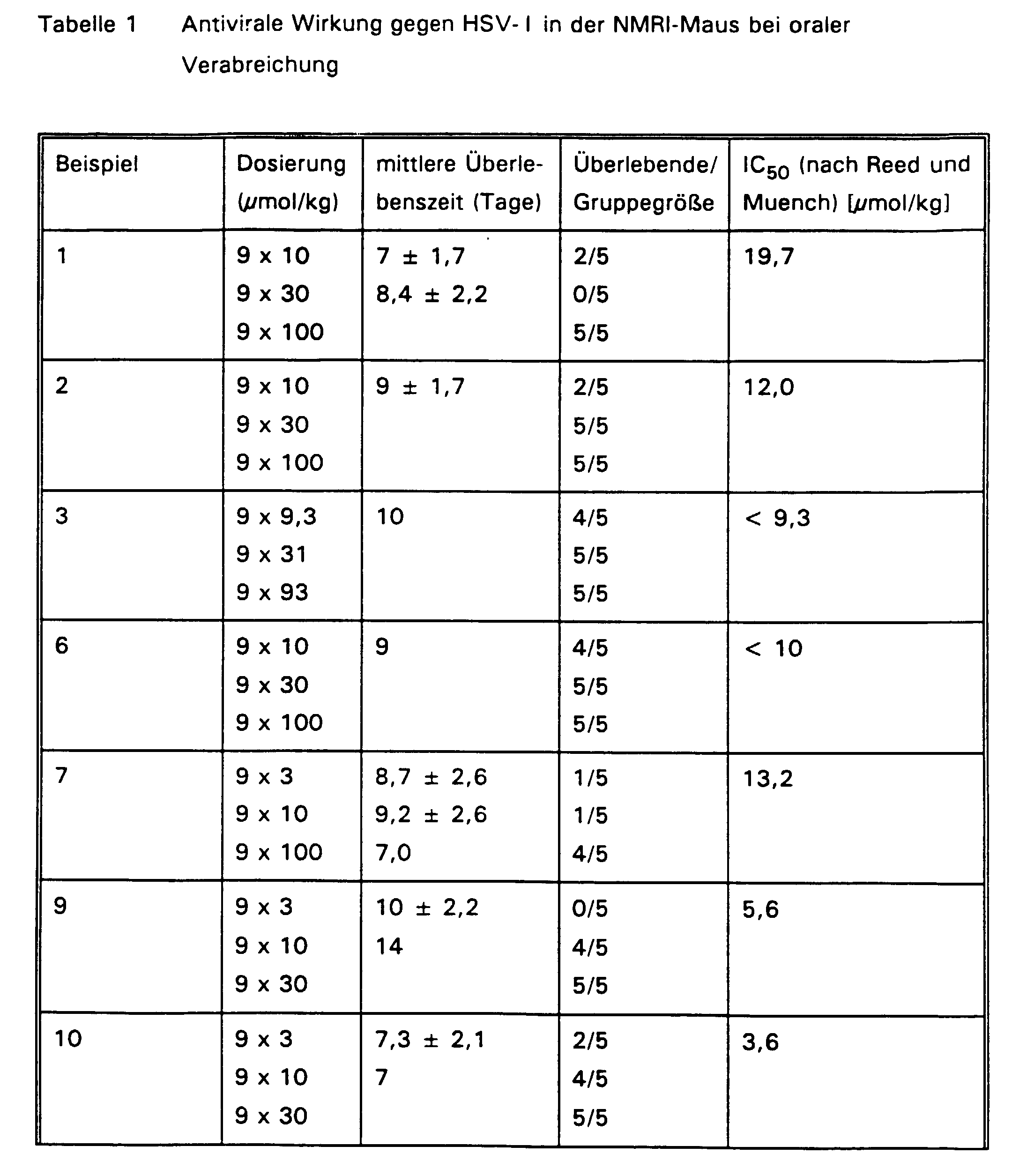

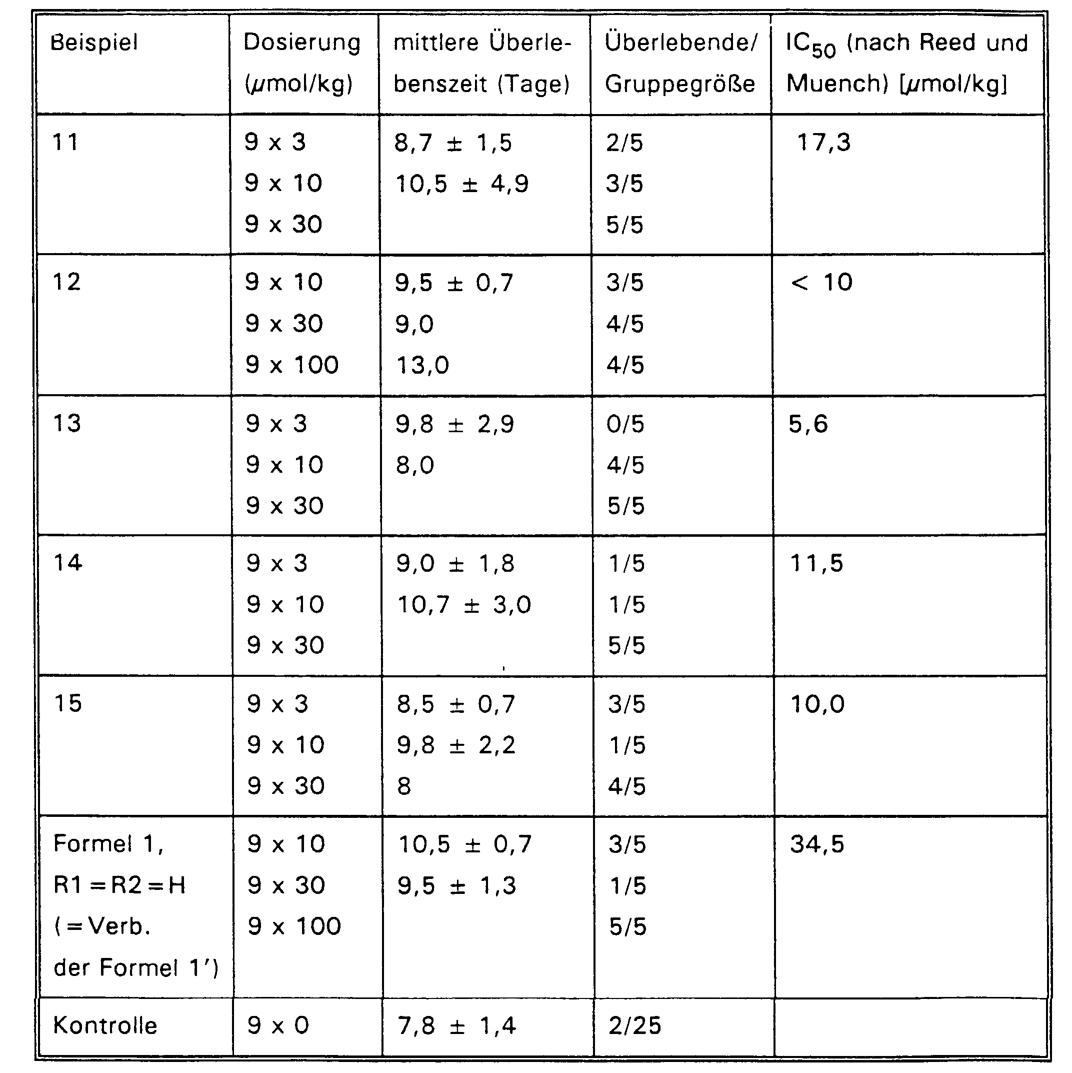

Durch die nachfolgenden Ausführungsbeispiele und durch den Inhalt der Patentansprüche wird die vorliegende Erfindung näher erläutert.

### Beispiele

1. Verbindung der Formel 1, wobei R¹ = R² = Pivaloyl ist;
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680 A beschrieben) werden in 50 ml wasserfreiem Dimethylformamid gelöst. Man gibt nacheinander 1,12 g (11 mMol) Pivalinsäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,56 g (5,5 mMol) Pivalinsäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 1,06 g (52,1 % d.Th.) 2-Amino-7-[(1,3-bis-pivaloyloxy-2-propoxy)methyl]purin mit dem Schmelzpunkt 191°C als farblose Kristalle.
   ¹H-NMR (270 Mhz, d₆-dmso) d [ppm]: 8,67 (s,1H), 8,45 (s,1H), 6,27 (s,2H), 5,70 (s,2H), 4,22 bis 3,90 (m,5H), 1,04 (s,18H).
2. Verbindung der Formel 1, wobei R¹ = R² = Valeroyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 1,2 ml (11 mMol) Valeriansäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,6 ml (5,5 mMol) Valeriansäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur.
   Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 1,71 g (84 % d.Th.) 2-Amino-7-[(1,3-bis-valeroyloxy-2-propoxy)methyl]purin mit dem Schmelzpunkt 91 bis 92°C als farblose Kristalle.
   ¹H-NMR (270 MHz, d₆-dmso) d [ppm]: 8,64 (s,1H), 8,41 (s,1H), 6,26 (s,2H), 5,67 (s,2H), 4,18 bis 3,90 (m,5H), 2,08 (t,4H), 1,37 (m,4H), 1,23 (m,4H), 0,83 (t,6H).
3. Verbindung der Formel 1, wobei R¹ = Wasserstoff und R² = Valeroyl ist:
   Aus dem im Beispiel 2 beschriebenen Ansatz lassen sich außerdem 0,22 g (13,6% d. Th.) des Monoesters 2-Amino-7-[(1-hydroxy-3-valeroyloxy-2-propoxy)methyl]purin mit dem Schmelzpunkt 95°C als farblose Kristalle gewinnen.
   ¹H-NMR (200 MHz, d₆-dmso) d [ppm]: 8,65 (s,1H), 8,40 (s,1H), 6,25 (s,2H), 5,68 (m,2H), 4,87 (t,1H), 4,15 bis 4,05 (m,1H), 3,95 bis 3,85 (m,1H), 3,75 bis 3,60 (m,1H), 3,50 bis 3,38 (m,2H), 2,0 (t,2H), 1,40 bis 1,10 (m,4H), 0,82 (t,3H).
4. Verbindung der Formel 1, wobei R¹ = Wasserstoff und R² = Oleoyl ist:
   1,5 g (6,3 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 3,74 g (13,2 mMol) Ölsäure, 0,23 g (1,89 mMol) N,N-Dimethylaminopyridin und 5,2 g (25,2 mMol)
   N,N'-Dicyclohexylcarbodiimid zu und rührt die Mischung 85 Stunden bei Raumtemperatur. Ausgefallener N,N'-Dicyclohexylharnstoff wird abfiltriert, das Filtrat im Vakuum eingeengt und der Rückstand über Kieselgel mit
   Essigsäureethylester/Methanol 9/1 als Laufmittelgemisch chromatographiert. Man erhält 0,5 g (15,8 % d.Th.) 2-Amino-7-[(1-hydroxy-3-oleoyloxy-2-propoxy)methyl]purin als glasartige Substanz.
   ¹H-NMR (200 MHz, d₆-dmsol d [ppm]: 8,63 (s,1H), 8,40 (s,1H), 6,23 (s,2H), 5,68 (m,2H), 5,32 (m,2H), 4,85 (s,breit,1H), 4,15 bis 3,83 (m,2H), 3,70 bis 3,62 (m,1H), 3,4 (m,2H), 2,05 bis 1,90 (m,6H), 1,45 bis 1,12 (m,22H), 0,88 (t,3H).
5. Verbindung der Formel 1, wobei R¹ = Wasserstoff und R² = Stearoyl ist:
   1,5 g (6,3 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 3,76 g (13,2 mMol) Octadecansäure, 0,23 g (1,89 mMol) N,N-Dimethylaminopyridin und 5,2 g (25,2 mMol) N,N'-Dicyclohexylcarbodiimid zu und rührt die Mischung 85 Stunden bei Raumtemperatur. Ausgefallener N,N'-Dicyclohexylharnstoff wird abfiltriert, das Filtrat im Vakuum eingeengt und der Rückstand über Kieselgel mit Essigsäureethylester/Methanol 9/1 als Laufmittelgemisch chromatographiert. Man erhält 1,6 g (50,3 % d.Th.) 2-Amino-7-[(1-hydroxy-3-stearoyloxy-2-propoxy)methyl]purin als glasartige Substanz.
   ¹H-NMR (200 MHz, d₆-dmso) d [ppm]: 8,62 (s,1H), 8,38 (s,1H), 6,23 (s,2H), 5,68 (m,2H), 4,85 (t,1H), 4,15 bis 3,83 (m,2H), 3,70 bis 3,62 (m,1H), 3,5 bis 3,35 (m,2H), 2,0 (t,2H), 1,80 bis 1,05 (m,30H), 0,85 (t,3H).
6. Verbindung der Formel 1, wobei R¹ = R² = Methoxyacetyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 0,85 ml (11 mMol) Methoxyessigsäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,42 ml (5,5 mMol) Methoxyessigsäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 1,13 g (59,1 % d.Th.) 2-Amino-7-[(1,3-bis-methoxyacetoxy-2-propoxy)methyl]purin mit dem Schmelzpunkt 86-87°C als farblose Kristalle.
   ¹H-NMR (270 MHz, d₆-dmso) d [ppm]: 8,66 (s,1H), 8,42 (s,1H), 6,31 (s,2H), 5,69 (s,2H), 4,27 bis 3,92 (m,5H), 3,84 (d,4H), 3,26 (s,6H).
7. Verbindung der Formel 1, wobei R¹ = R² = Ethoxyacetyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 1,05 ml (11 mMol) Ethoxyessigsäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,53 ml (5,5 mMol) Valeriansäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 0.79 g (38,4 % d.Th.) 2-Amino-7-[(1,3-bis-ethoxyacetoxy-2-propoxy)methyl]purin mit dem Schmelzpunkt 78 bis 79°C als farblose Kristalle.
   ¹H-NMR (270 MHz, d₆-dmso) d [ppm]: 8,64 (s,1H), 8,41 (s,1H), 6,27 (s,2H), 5,66 (s,2H), 4,25 bis 3,91 (m,5H), 3,85 (s,4H), 3,41 (q,4H), 1,10 (t,6H),
8. Verbindung der Formel 1, wobei R¹ = Wasserstoff und R² = Ethoxyacetyl ist:
   Aus dem im Beispiel 7 beschriebenen Ansatz lassen sich außerdem 0,63 g (38,8% d. Th.) des Monoesters 2-Amino-7-[(1-ethoxyacetoxy-3-hydroxy-2-propoxy)methyl]purin mit dem Schmelzpunkt 97 bis 98°C als farblose Kristalle gewinnen.
   ¹H-NMR (270 MHz, d₆-dmso) d [ppm]: 8,65 (s,1H), 8,41 (s,1H), 6,25 (s,2H), 5,66 (s,2H), 4,87 (t,1H), 4,21 bis 3,90 (m,2H), 3,79 (s,4H), 3,68 (m,1H), 3,43 bis 3,35 (m,4H), 1,09 (t,3H).
9. Verbindung der Formel 1, wobei R¹ = R² = Hexahydrobenzoyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 1,41 g (11 mMol) Cyclohexancarbonsäure, 0,2 g (1,6 mMol) N,N-Dimethylaminopyridin und 3,96 g (19 mMol) N,N'-Dicyclohexylcarbodiimid zu und rührt die Mischung 4 Stunden bei 50°C. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 0,65 g (28,3 % d.Th.) 2-Amino-7-[(1,3-bis-cyclohexancarbonyloxy-2-propoxy)methyl]purin mit dem Schmelzpunkt 150°C als farblose Kristalle.
   ¹H-NMR (270 MHz, d₆-dmso) d [ppm]: 8,64 (s,1H), 8,41 (s,1H), 6,25 (s,2H), 5,66 (s,2H), 4,20 bis 3,89 (m,5H), 2,15 bis 1,90 (m,2H), 1,65 bis 1,45 (m,10H), 1,35 bis 1,05 (m,10H).
10. Verbindung der Formel 1, wobei R¹ = Wasserstoff und R² =
   Hexahydrobenzoyl ist:
   Aus dem im Beispiel 9 beschriebenen Ansatz lassen sich außerdem 0,38 g (21,8% d. Th.) des Monoesters 2-Amino-7-[(1-cyclohexancarbonyloxy-3-hydroxy-2-propoxy)methyl] purin mit dem Schmelzpunkt 128°C als farblose Kristalle gewinnen. ¹H-NMR (270 MHz, d₆-dmso) d [ppm]: 8,63 (s,1H), 8,39 (s,1H), 6,25 (s,2H), 5,66 (s,2H), 4,88 (t,1H), 4,15-3,82 (m,2H), 3,75 bis 3,60 (m,1H), 3,50 bis 3,35 (m,2H), 1,95 bis 1,80 (m,1H), 1,70 bis 1,45 (m,5H), 1,30 bis 1,0 (m,5H).
11. Verbindung der Formel 1, wobei R¹ = R² = Benzoyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 1,34 g (11 mMol) Benzoesäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,67 g (5,5 mMol) Benzoesäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 1,07 g (47,9 % d.Th.) 2-Amino-7-[(1,3-bis-benzoyloxy-2-propoxy)methyl]purin mit dem Schmelzpunkt 147 bis 148°C als farblose Kristalle.
   ¹H-NMR (270 MHz, d₆-dmso) d [ppm]: 8,70 (s,1H), 8,51 (s,1H), 7,80 bis 7,44 (m,10H), 6,27 (s,2H), 5,81 (s,2H), 4,65 bis 4,55 (m,2H), 4,43 bis 4,24 (m,3H).
12. Verbindung der Formel 1, wobei R¹ = R² = Phenylacetyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 1,5 g (11 mMol) Phenylessigsäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,75 g (5,5 mMol) Phenylessigsäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 1,20 g (50,5 % d.Th.) 2-Amino-7-[(1,3-bis-phenylacetoxy-2-propoxy)methyl]purin mit dem Schmelzpunkt 94 bis 95°C als farblose Kristalle.
   ¹H-NMR (270 MHz, d₆-dmso) d [ppm]: 8,69 (s,1H), 8,41 (s,1H), 7,35 bis 7,15 (m,10H), 6,31 (s,2H), 5,63 (s,2H), 4,20 bis 3,95 (m,5H), 3,46 (s,4H).
13. Verbindung der Formel 1, wobei R¹ = R² = Thiophen-3-carbonyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 1,44 g (11 mMol) Thiophen-3-carbonsäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,72 ml (5,5 mMol) Thiophen-3-carbonsäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 0,31 g (13,5 % d.Th.) 2-Amino-7[(1,3-bis-(thiophen-3-carbonyloxyl-2-propoxy)methyl]purin mit dem Schmelzpunkt 168°C als farblose Kristalle.
   ¹H-NMR (200 MHz, d₆-dmso) d [ppm]: 8,66 (s,1H), 8,47 (s,1H), 8,14 (m,2H), 7,63 (m,2H), 7,28 (m,2H), 6,25 (s,2H), 5,67 (s,2H), 4,55 bis 4,43 (m,2H), 4,35 bis 4,15 (m,3H).
14. Verbindung der Formel 1, wobei R¹ = Wasserstoff und R² = Thiophen-3-carbonyl ist:
   Aus dem im Beispiel 13 beschriebenen Ansatz lassen sich außerdem 0,62 g (35,5% d. Th.) des Monoesters 2-Amino-7-[(1-hydroxy-3-(thiophen-3-carbonyloxyl-2-propoxylmethyl]purin mit dem Schmelzpunkt 141-143°C als farblose Kristalle gewinnen.
   ¹H-NMR (200 MHz, d₆-dmso) d [ppm]: 8,66 (s,1H), 8,43 (s,1H), 8,09 (m,1H), 7,6 (m,1H), 7,25 (m,1H), 6,24 (s,2H), 5,72 (s,2H), 4,89 (t,1H), 4,40 bis 4,30 (m,1H), 4,20 bis 4,05 (m,1H), 3,90 bis 3,75 (m,1H), 3,60 bis 3,40 (m,2H).
15. Verbindung der Formel 1, wobei R¹ = R² = 3-Pyridylacetyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 1,90 g (11 mMol)
   3-Pyridylessigsäure Hydrochlorid, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,95 g (5,5 mMol) 3-Pyridylessigsäure Hydrochlorid, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 5/1 als Laufmittelgemisch chromatographiert. Man erhält 2,12 g (88,9 % d.Th.) 2-Amino-7-[(1,3-bis-(3-pyridylacetoxy)-2-propoxylmethyl]purin als glasartige Substanz.
   ¹H-NMR (200 MHz, d₆-dmso) d [ppm]: 8,69 (s,1H), 8,44 (m,5H), 7,61 (m,2H), 7,33 (m,2H), 6,31 (s,2H), 5,64 (s,2H), 4,23 bis 3,93 (m,5H), 3.54 (s,4H).

## Patentansprüche

1. Verbindungen der Formel 1 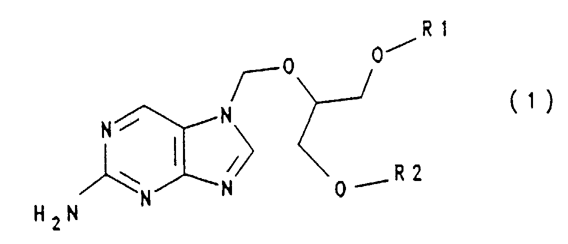 in denen die Reste R¹ und/oder R² unabhängig voneinander Acylreste der Formel
-C(=O)-R³ (2)
darstellen,
wobei R³ C1-C5-Alkoxymethyl, C4-C17-Alkyl, C6-C17-Alkenyl, C3-C8-Cycloalkyl, Aryl mit 6-14 C-Atomen, vorzugsweise 6 C-Atomen gegebenenfalls substituiert mit C1-C3-Alkyl oder Methoxy; Heteroaryl mit 3 bis 10 C-Atomen, vorzugsweise 4 oder 5 C-Atomen, mit bis zu 3 Heteroatomen, ausgewählt aus der Gruppe O, N, und S, vorzugsweise mit einem oder zweien, insbesondere einem O- oder N-Atom; Arylmethyl mit Aryl wie vorstehend definiert, Heteroarylmethyl mit Heteroaryl wie vorstehend definiert bedeutet und einer der Reste R¹ und R² auch Wasserstoff bedeuten kann,
sowie deren physiologisch verträgliche Salze.

2. Verbindungen der Formel 1 gemäß Anspruch 1, dadurch gekennzeichnet, daß R³ C1-C3-Alkoxymethyl, C4-C17-Alkyl, C13-C17-Alkenyl, C5-C6-Cycloalkyl, Phenyl, Thiophen-3-yl, Benzyl oder Pyridin-3-yl-methyl bedeutet und einer der Reste R¹ und R² auch Wasserstoff bedeuten kann.

3. Verbindungen der Formel 1 gemäß Ansprüchen 1 oder 2, dadurch gekennzeichnet, daß R³ C1-C2-Alkoxymethyl, C17-Alkyl, C17-Alkenyl, C6-Cycloalkyl, Phenyl, Thiophen-3-yl, Benzyl oder Pyridin-3-yl-methyl bedeutet und einer der Reste R¹ und R² auch Wasserstoff bedeuten kann.

4. Verfahren zur Herstellung von Verbindungen der Formel 1 gemäß den Ansprüchen 1 bis 3, dadurch gekennzeichnet, daß man die Verbindung der Formel 1', 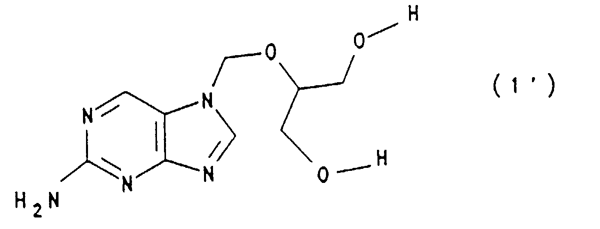 mit Carbonsäuren der Formel 3,
R⁴-COOH (3)
wobei R⁴ C1-C5-Alkoxymethyl, C4-C17-Alkyl, C6-C17-Alkenyl, C3-C8-Cycloalkyl, Aryl, Heteroaryl, Arylmethyl oder Heteroarylmethyl wobei Aryl, Heteroaryl, Arylmethyl und Heteroarylmethyl wie in Anspruch 1 definiert sind, bedeutet, mit einem wasserentziehenden Mittel wie N,N'-Dicyclohexylcarbodiimid unter Beifügung einer Base wie N,N-Dimethylaminopyridin in einem Lösungsmittel wie Dimethylformamid unter Erhalt von Verbindungen der Formel 1 umsetzt, und anschließend die Mono- und Diester voneinander mittels herkömmlicher Methoden isoliert.

5. Verbindungen der Formel 1 gemäß den Ansprüchen 1 bis 3 zur Anwendung als antivirales Mittel.

6. Verbindungen der Formel 1 gemäß Ansprüchen 1 bis 3 zur Anwendung als Antiherpesmittel.

7. Arzneimittel mit einem Gehalt an mindestens einer Verbindung der Formel 1 gemäß den Ansprüchen 1 bis 3.

8. Verwendung von Verbindungen der Formel 1 gemäß den Ansprüchen 1 bis 3 zur Herstellung von Arzneimitteln zur Behandlung von Viruserkrankungen.

9. Verfahren zur Herstellung von Arzneimitteln gemäß Anspruch 7, dadurch gekennzeichnet, daß man mindestens eine Verbindung der Formel 1 gemäß den Ansprüchen 1 bis 3 gegebenenfalls mit geeigneten Hilfs- und/oder Trägerstoffen in eine geeignete Darreichungsform bringt.

## Claims

1. A compound of the formula 1 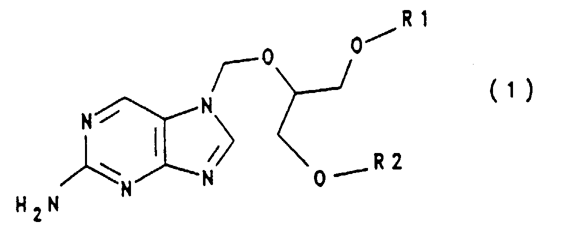 in which the radicals R¹ and/or R², independently of each other, are acyl radicals of the formula
-C(=O)-R³ (2)
where R³ is C1-C5-alkoxymethyl, C4-C17-alkyl, C6-C17-alkenyl, C3-C8-cycloalkyl, aryl having 6-14 carbon atoms, preferably 6 carbon atoms, optionally substituted by C1-C3-alkyl or methoxy; heteroaryl having 3 to 10 carbon atoms, preferably 4 or 5 carbon atoms, having up to 3 heteroatoms, selected from the group consisting of O, N and S, preferably having one or two, in particular one, O or N atom; arylmethyl where aryl is as defined above, heteroarylmethyl where heteroaryl is as defined above, and one of the radicals R¹ and R² can also be hydrogen, as well as physiologically tolerated salts thereof.

2. A compound of the formula 1 as claimed in claim 1, wherein R³ is C1-C3-alkoxymethyl, C4-C17-alkyl, C13-C17-alkenyl, C5-C6-cycloalkyl, phenyl, thiophen-3-yl, benzyl or pyridin-3-ylmethyl, and one of the radicals R¹ and R² can also be hydrogen.

3. A compound of the formula 1 as claimed in claims 1 or 2, wherein R³ is C1-C2-alkoxymethyl, C17-alkyl, C17-alkenyl, C6-cycloalkyl, phenyl, thiophen-3-yl, benzyl or pyridin-3-ylmethyl, and one of the radicals R¹ and R² can also be hydrogen.

4. A process for preparing compounds of the formula 1 as claimed in claims 1 to 3, wherein the compound of the formula 1', 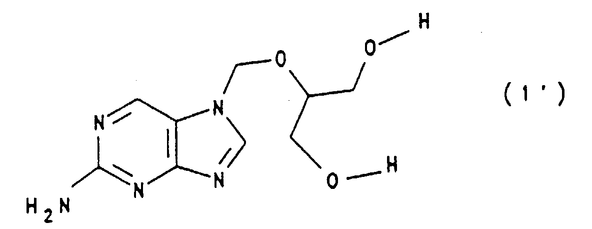 is reacted with carboxylic acids of the formula 3,
R⁴-COOH (3)
where R⁴ is C1-C5-alkoxymethyl, C4-C17-alkyl, C6-C17-alkenyl, C3-C8-cycloalkyl, aryl, heteroaryl, arylmethyl or heteroarylmethyl, aryl, heteroaryl, arylmethyl and heteroarylmethyl being as defined in claim 1, together with a dehydrating agent such as N,N'-dicyclohexylcarbodiimide and with the addition of a base, such as N,N-dimethylaminopyridine, in a solvent such as dimethylformamide, to produce compounds of the formula 1, and the monoesters and diesters are subsequently isolated from each other using conventional methods.

5. A compound of the formula 1 as claimed in claims 1 to 3 for use as an antiviral agent.

6. A compound of the formula 1 as claimed in claims 1 to 3 for use as an anti-herpes agent.

7. A pharmaceutical containing at least one compound of the formula 1 as claimed in claims 1 to 3.

8. The use of compounds of the formula 1 as claimed in claims 1 to 3 for preparing pharmaceuticals for treating viral diseases.

9. A process for preparing pharmaceuticals as claimed in claim 7, wherein at least one compound of the formula 1 as claimed in claims 1 to 3 is converted, where appropriate together with suitable auxiliary substances and/or excipients, into a suitable form for administration.

## Revendications

1. Composés de formule 1 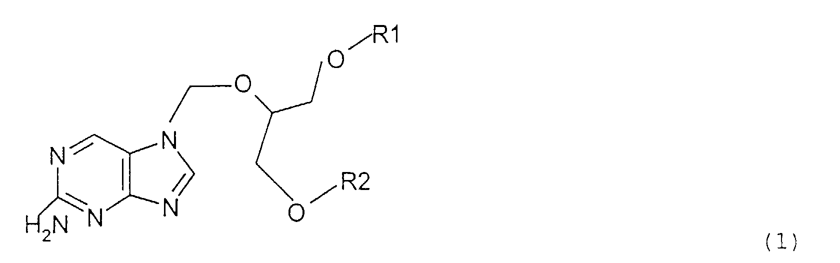 dans lesquels les restes R¹ et/ou R² représentent, indépendamment l'un de l'autre, des restes acyle de formule
- C (=O) - R³ (2)
dans laquelle R³ représente un groupe alcoxyméthyle en C₁-C₅, alkyle en C₄-C₁₇, alcényle en C₆-C₁₇, cycloalkyle en C₃-C₈, aryle comportant de 6 à 14 atomes de carbone, de préférence 6 atomes de carbone, éventuellement substitué par un groupe alkyle ou méthoxy en C₁-C₃; un groupe hétéroaryle comportant de 3 à 10 atomes de carbone, de préférence 4 ou 5 atomes de carbone, comportant jusqu'à 3 hétéroatomes, choisis dans le groupe formé par O, N et S, de préférence comportant un ou deux hétéroatomes, en particulier un atome O et un atome N; un groupe arylméthyle avec le groupe aryle tel que défini précédemment, un groupe hétéroarylméthyle avec le groupe hétéroaryle tel que défini précédemment et l'un des restes R¹ et R² peut représenter aussi un atome d'hydrogène, ainsi que leurs sels physiologiquement acceptables.

2. Composés de formule 1, selon la revendication 1, caractérisés en ce que R³ peut représenter un groupe alcoxyméthyle en C₁-C₃, alkyle en C₄-C₁₇, alcényle en C₁₃-C₁₇, cycloalkyle en C₅-C₆, phényle, thiophène-3-yle, benzyle ou pyridine-3-yl-méthyle et l'un des restes R¹ et R² peut représenter aussi un atome d'hydrogène.

3. Composés de formule 1, selon les revendications 1 ou 2, caractérisés en ce que R³ peut représenter un groupe alcoxyméthyle en C₁-C₂, alkyle en C₁₇, alcényle en C₁₇, cycloalkyle en C₆, phényle, thiophène-3-yle, benzyle ou pyridine-3-yl-méthyle et l'un des restes R¹ et R² peut représenter aussi un atome d'hydrogène.

4. Procédé de préparation de composés de formule 1 selon les revendications 1 à 3, caractérisé en ce que l'on convertit le composé 1', 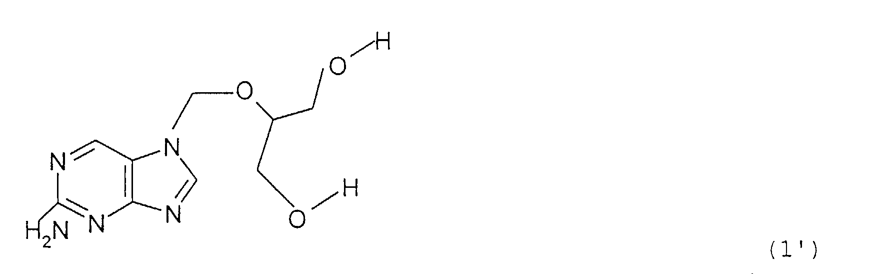 avec des acides carboxyliques de formule 3
R⁴ - COOH (3)
dans laquelle R⁴ représente un groupe alcoxyméthyle en C₁-C₅, alkyle en C₄-C₁₇, alcényle en C₆-C₁₇, cycloalkyle en C₃-C₈, aryle, hétéroaryle, arylméthyle ou hétéroarylméthyle, dans lequel les groupes aryle, hétéroaryle, arylméthyle et hétéroarylméthyle sont définis comme dans la revendication 1, à l'aide d'un agent capable de piéger l'eau tel que du N,N'-dicyclohexylcarbodiimide, avec addition d'une base telle que de la N,N-diméthylaminopyridine, dans un solvant tel que du diméthylformamide, pour obtenir des composés de formule 1, puis on isole les mono- et diesters les uns des autres, à l'aide de méthodes classiques.

5. Composés de formule 1, selon les revendications 1 à 3, pour l'application comme agent antiviral.

6. Composés de formule 1, selon les revendications 1 à 3, pour l'application comme agent antiherpès.

7. Médicaments contenant -au-moins un des composés de formule 1, selon les revendications 1 à 3.

8. Utilisation de composés de formule 1 selon les revendications 1 à 3 pour la préparation de médicaments destinés au traitement de maladies virales.

9. Procédé de préparation de médicaments selon la revendication 7, caractérisé en ce qu'on formule au moins un composé de formule 1, selon les revendications 1 à 3, éventuellement avec des substances auxiliaires et/ou des véhicules appropriés sous une forme d'administration appropriée.
